Europäisches Patentamt

European Patent Office

Office européen des brevets

(11) Veröffentlichungsnummer: **0 471 203 A2**

# EUROPÄISCHE PATENTANMELDUNG

(21) Anmeldenummer: **91112065.7**

(22) Anmeldetag: **18.07.91**

(51) Int. Cl.⁵: **G01N 33/543**, //B08B3/08, B08B9/28

(30) Priorität: **06.08.90 DE 4024932**

(43) Veröffentlichungstag der Anmeldung: **19.02.92 Patentblatt 92/08**

(84) Benannte Vertragsstaaten:
**AT BE CH DE DK ES FR GB GR IT LI LU NL SE**

(71) Anmelder: **HENNING BERLIN GmbH CHEMIE-UND PHARMAWERK**
**Komturstrasse 58-62**
**W-1000 Berlin 42(DE)**

(72) Erfinder: **Bergmann, Andreas, Dr.**
**Baumläuferweg 47**
**W-1000 Berlin 47(DE)**

(74) Vertreter: **Andrae, Steffen, Dr. et al**
**Patentanwälte Andrae, Flach, Haug, Kneissl**
**Balanstrasse 55**
**W-8000 München 90(DE)**

(54) **Verfahren zum Waschen von Trägern mit immobilisierten Reaktionsprodukten oder Reaktionspartnern, die bei immundiagnostischen Bestimmungsverfahren gebildet oder verwendet werden, sowie Vorrichtung zur Durchführung dieses Verfahrens.**

(57) Mit bei immundiagnostischen Bestimmungsverfahren gebildeten immobilisierten Immunkomplexen oder mit für die Verwendung in immundiagnostischen Bestimmungsverfahren vorgesehenen immobilisierten Reaktionspartnern belegte feste Träger werden so gewaschen, daß die Waschflüssigkeit während des Waschens frei abfließen kann. Das wird vorzugsweise dadurch erreicht, daß die Träger wie Teströhrchen mit ihren Öffnungen nach unten angeordnet und mit einem Flüssigkeitsnebel ausgesprüht werden.

Eine Vorrichtung zur Durchführung eines solchen Verfahrens besteht aus einer drehbaren Haltevorrichtung (1) und einer Sprühvorrichtung (4) mit aufwärts gerichteten Sprühdüsen (5).

Fig. 1

Die Erfindung betrifft ein Verfahren zum Waschen von Trägern mit immobilisierten Reaktionsprodukten oder Reaktionspartnern, die bei immundiagnostischen Bestimmungsverfahren gebildet oder verwendet werden, sowie Vorrichtungen zur Durchführung eines solchen Verfahrens.

Insbesondere betrifft die Erfindung ein Verfahren zur Durchführung der Waschschritte bei einem immundiagnostischen Verfahren zur Konzentrationsbestimmung von Biomolekülen wie Antikörpern, Antigenen und Haptenen in flüssigen Medien unter Verwendung von mit immobilisierten spezifischen Bindungspartnern für diese Biomoleküle oder mit immobilisierten Formen dieser Biomoleküle beschichteten festen Trägern sowie Vorrichtungen zur Durchführung eines solchen Verfahrens.

Immundiagnostische Verfahren (immunoassays) der eingangs genannten Art lassen sich ganz allgemein so charakterisieren, daß man bei ihnen flüssige Medien, insbesondere biologische Flüssigkeiten, die die zu bestimmenden Biomoleküle sowie gegebenenfalls gelöste oder suspendierte spezifische Bindungspartner für diese Biomoleküle enthalten, in einem oder mehreren Schritten mit beschichteten Trägern in Berührung bringt, deren Oberflächen je nach Art der zu bestimmenden Substanz und des angewendeten immundiagnostischen Bestimmungsverfahrens mit immobilisierten spezifischen Bindungspartnern für diese Biomoleküle oder mit immobilisierten Formen, z.B. Derivaten, dieser Biomoleküle belegt oder beschichtet sind, daß man nach einer dem jeweiligen Bestimmungsverfahren angepaßten Inkubationszeit Träger und flüssige Medien voneinander trennt, die Träger wäscht und anschließend die Mengen der bei der Inkubation gebildeten Immunkomplexe anhand geeigneter physikalischer Markierungen oder anhand geeigneter Folgereaktionen bestimmt und daraus die Konzentrationen der Biomoleküle in den flüssigen Medien errechnet.

Bei Verfahren, die nach dem sogenannten Doppelantikörperverfahren arbeiten, werden die Immunkomplexe auch durch eine weitere Umsetzung der Träger mit einem Markierungsmolekül (einem zweiten markierten Antikörper) umgesetzt, und die Mengen der immobilisierten Immunkomplexe werden dann nach einer Wiederholung der Trenn- und Waschschritte anhand der Markierungen der zweiten Antikörper ermittelt.

Zur Durchführung derartiger Bestimmungsverfahren werden kommerziell Immundiagnostika angeboten, wobei bei einem Großteil derartiger Immundiagnostika als feste Träger Röhrchen, in der Regel leichte Kunststoff-Teströhrchen, oder Mikrotiterplatten verwendet werden, die mit den spezifischen Bindungspartnern wie Antigenen, Antikörpern oder Rezeptoren beschichtet sind. Die Feststellung des Ausmaßes der Bindung der zu bestimmenden Biomoleküle erfolgt über an sich bekannte Tracer verschiedener Art, zu denen fluoreszierende, radioaktive, chemilumineszierende oder enzymatisch aktive Tracer gehören. Die verschiedenen Verfahrensvarianten werden in den einschlägigen Lehrbüchern mit Kurzbezeichnungen wie IRMA, ILMA, EIA, ELISA, SPART, SPALT, Coated Tube-RIA, LIA usw. bezeichnet.

Wenn die zur Bildung immobilisierter Immunkomplexe führenden Umsetzungen abgeschlossen sind, muß der feste Träger von dem flüssigen Medium und somit dem darin enthaltenen Tracer getrennt werden, was in aller Regel durch Absaugen der flüssigen Phase aus dem Teströhrchen oder aus den Vertiefungen der Mikrotiterplatten erfolgt. Damit die Menge des im Teströhrchen oder auf der Mikrotiterplatte verbliebenen Tracers tatsächlich ein exaktes Maß für die unbekannte Konzentration des zu bestimmenden Biomoleküls ist, muß die Trennung der an der festen Phase immobilisierten Moleküle von den ungebundenen Molekülen in der flüssigen Phase eine möglichst vollständige sein. Zu diesem Zwecke werden die festen Träger, also insbesondere die Teströhrchen und Mikrotiterplatten, nach der Entfernung der Testlösung noch mit einer Waschlösung gewaschen. Die Waschlösung oder Waschflüssigkeit enthält dabei üblicherweise an sich bekannte Zusätze, die eine Beeinträchtigung der Protein-Protein-Wechselwirkung, auf der die Bindung der zu bestimmenden Biomoleküle an die immobilisierten Moleküle beruht, vermeidet. Derartige an sich bekannte Zusätze sind Salz- bzw. Pufferlösungen, insbesondere physiologische Salzlösungen, geeignete Detergenslösungen oder Lösungen, die sowohl Salze bzw. Puffer und Detergenzien enthalten. Die Möglichkeit einer Verwendung derartiger Waschlösungen ist im Rahmen der vorliegenden Anmeldung auch dann stets vorgesehen, wenn nur von "Spülwasser" gesprochen wird.

Für dieses Waschen sind im Handel spezielle mehr oder weniger automatisierte Waschgeräte erhältlich, bei denen der Waschschritt ausnahmslos so durchgeführt wird, daß die aufrechtstehenden Röhrchen mit einer Waschlösung gefüllt werden, und nach einer vorgegebenen kürzeren oder längeren Zeit wird diese Waschlösung dekantiert oder abgesaugt. Je nach Bedarf wird dieser Waschschritt mehrmals wiederholt. Bei der Vielzahl der im Handel erhältlichen Waschgeräte wird dieser Waschvorgang automatisch durchgeführt. Er kann jedoch auch entsprechend manuell durchgeführt werden.

Es hat sich nunmehr gezeigt, daß die Art der Waschung der beschichteten festen Träger nicht nur für die für eine Untersuchung benötigte Zeitdauer von Bedeutung ist, sondern daß auch die Präzision und Fehlerbreite der erhaltenen Meßergebnisse erheblich durch den Waschvorgang beeinflußt wird.

Es ist Aufgabe der vorliegenden Erfindung, die Präzision eines immundiagnostischen Verfahrens zur Konzentrationsbestimmung von Biomolekülen wie Antikörpern, Antigenen und Haptenen in flüssigen Medien unter Verwendung von mit immobilisierten spezifischen Bindungspartnern für diese Biomoleküle oder mit immobilisierten Formen dieser Biomoleküle beschichteten festen Trägern zu verbessern, ohne daß der erforderliche Zeitaufwand erhöht wird.

Es ist ferner Aufgabe der vorliegenden Erfindung, eine Vorrichtung zur Durchführung eines derartigen verbesserten Verfahrens zu schaffen.

Diese Aufgaben werden bei einem Verfahren gemäß Oberbegriff des Patentanspruchs 1 dadurch gelöst, daß man die Träger in dem oder den Waschschritt(en) so mit der Waschflüssigkeit behandelt, daß die Waschflüssigkeit während des gesamten Waschvorgangs frei von den Trägern abfließen kann.

Vorteilhafte Ausgestaltungen eines solchen Verfahrens sind den Unteransprüchen zu entnehmen.

Eine Vorrichtung zur Durchführung eines erfindungsgemäßen Verfahrens ist durch die Merkmale des Kennzeichens von Anspruch 8 gekennzeichnet. Vorteilhafte Ausgestaltungen sind den Unteransprüchen 9 bis 23 zu entnehmen.

Das erfindungsgemäße Verfahren beruht auf der überraschenden Feststellung, daß wesentlich bessere Ergebnisse als beim konventionellen Waschen von Teströhrchen oder Mikrotiterplatten mit aufrechtstehenden Röhrchen dann erhalten werden, wenn diese während des Waschens so mit der Waschflüssigkeit behandelt werden, daß die Waschflüssigkeit während des Waschens frei von den Trägern mit den immobilisierten Immunkomplexen abfließen kann, was insbesondere dadurch erreicht wird, daß man die Träger in Form von Teströhrchen oder Mikrotiterplatten mit ihren Öffnungen nach unten anordnet und die Flächen, an die die immobilisierten Immunkomplexe gebunden sind, mit einem Flüssigkeitsstrahl oder -nebel absprüht. Es wurde festgestellt, daß beim Ausspülen von auf dem Kopf stehenden Röhrchen bei einer Spülflüssigkeits-Durchflußgeschwindigkeit von 2 ml/s/Röhrchen eine Spüldauer von nur 5 s weitaus bessere Ergebnisse erbringt als ein dreimaliges Waschen nach der herkömmlichen Arbeitsweise, für das eine Dauer von etwa 30 s erforderlich ist. Das erfindungsgemäße Verfahren führt somit zu einer erheblichen Verkürzung der für die Bestimmung erforderlichen Zeitdauer.

Außerdem erhöht sich auch die Qualität der Bestimmung, was sich beispielsweise an der Verringerung der bei den einzelnen Messungen erhaltenen Standardabweichungen und damit der Fehlerbreite zeigt.

Das erfindungsgemäße Verfahren kann mit Vorteil ferner auch dann angewandt werden, wenn man im Rahmen der Herstellung der beschichteten Träger, die später bei dem immundiagnostischen Bestimmungsverfahren verwendet werden sollen, die frisch mit den spezifischen Bindungspartnern, insbesondere Antikörpern, oder den immobilisierten Formen der zu bestimmenden Biomoleküle beschichteten, ursprünglich neutralen aktivierten Träger zur Entfernung von nicht gebundenen Molekülen bzw. Resten der Behandlungslösung wäscht. Neben einer auch in einem solchen Falle feststellbaren Verminderung des Zeitaufwands für die Waschschritte fördert die Identität der Waschverfahren bei der Trägerherstellung und der späteren Bestimmung der Mengen der gebundenen Immunkomplexe auch noch die Präzision der Bestimmung.

Zur Durchführung des erfindungsgemäßen Verfahrens lassen sich grundsätzlich alle Vorrichtungen verwenden, die es ermöglichen, Mikrotiterplatten oder insbesondere Teströhrchen sicher mit ihren Öffnungen nach unten anzuordnen und diese dann von unten aus einer geeigneten Sprühvorrichtung mit der jeweiligen Waschflüssigkeit auszuspülen.

Es ist vorteilhaft, wenn derartige Vorrichtungen so ausgebildet sind, daß die Teströhrchen oder Mikrotiterplatten in Normallage in einer geeigneten Haltevorrichtung angeordnet werden können und dann zusammen mit dieser Haltevorrichtung mit ihren Öffnungen nach unten über die Sprühvorrichtungen gedreht und in dieser Stellung fixiert werden können. So sind Röhrchenhalterungen oder Röhrchengestelle denkbar, die in Normalstellung befüllt werden und dann frei oder durch Drehung um eine feste waagerechte Drehachse in die Spülstellung verschwenkt werden können. Das Verschwenken kann auch durch Verschieben der Haltevorrichtung auf einer Kreisbahn in geeigneten Führungen erfolgen.

Es kann vorteilhaft sein, zur Verbesserung der Präzision des Spülvorgangs die Spülvorrichtungen in einer festen räumlichen Beziehung zu den Halterungen der Teströhrchen und damit zu den Teströhrchen anzuordnen. Das kann beispielsweise dadurch erfolgen, daß die Spülvorrichtungen fest mit der Halterungsvorrichtung verbunden sind und vor der Einleitung des Spül- und Waschschritts mitverschwenkt werden und dabei unter die zu waschenden Teströhrchen geraten.

Das erfindungsgemäße Verfahren kann mit Vorteil mit Hilfe einer Vorrichtung verwirklicht werden, bei der die Anzahl der Spülvorrichtungen der Anzahl der zu waschenden Teströhrchen entspricht. Es ist jedoch grundsätzlich ohne weiteres auch möglich, in der Vorrichtung eine größere Anzahl von Teströhrchen fest anzuordnen und mit einer oder mehreren beweglichen Sprühvorrichtungen diese nacheinander zu waschen.

Vorzugsweise weisen Vorrichtungen zur Durchführung des erfindungsgemäßen Verfahrens aus Gründen der Arbeitssicherheit (Vermeidung radioaktiver Verschmutzung) und aus hygienischen Gründen Einrichtungen zum Sammeln des ablaufenden Waschwassers auf. Im einfachsten Falle kann das Waschwasser unter den ausgespülten Röhrchen in einer gemeinsamen rinnenartigen Vertiefung gesammelt und abgeleitet werden.

4

Um insbesondere bei Mikrotiterplatten zu vermeiden, daß ablaufendes Waschwasser aus einer Vertiefung der Mikrotiterplatte beispielsweise durch einen benachbarten Spülstrahl in eine benachbarte Vertiefung gerissen wird, kann es vorteilhaft sein, den Waschvorgang so durchzuführen, daß sichergestellt ist, daß das Waschwasser aus jedem einzelnen Teströhrchen bzw. jeder einzelnen Vertiefung einer Mikrotiterplatte sauber und ungestört ablaufen kann. Dieses getrennt ablaufende Waschwasser kann später zu einem einzigen Abwasserstrom vereinigt werden. Eine günstige apparative Möglichkeit zur Durchführung der Waschung besteht darin, die Sprühdüsen mit einer Ableitung für das Waschwasser zu kombinieren. Das kann beispielsweise dadurch geschehen, daß eine Sprühdüse, die bis ins Innere der Teströhrchen hineinreicht, in ihrem hinteren Teil konzentrisch von einer Waschwasserableitung umgeben wird, die an den Randbereich des Teströhrchens oder der Vertiefung einer Mikrotiterplatte zur Anlage gebracht werden kann. Beispielsweise kann dazu der Rand dieses Ableitungsröhrchens eine geeignete Gummidichtung oder -manschette aufweisen, die eine weiche, stoßfreie und doch dichte Anlage ermöglicht. In diesem Falle fließt das gesamte von der Sprühdüse im Inneren des Teströhrchens abgegebene Waschwasser frei nach unten ab und wird vollständig in einer Ableitung gesammelt. Je nach den möglichen Inhaltsstoffen und deren Gefahrenklasse kann das Abwasser einfach abgeleitet werden oder einer speziellen erforderlichen Reinigungsbehandlung zugeführt werden.

Es ist jedoch auch möglich, soweit nötig, spezielle Teströhrchen oder Mikrotiterplatten zu verwenden, die durch ihre Form einen störungsfreien Ablauf der Waschlösung gewährleisten. So sind beispielsweise Teströhrchen, deren Ränder in Zacken oder dünnen Fortsätzen auslaufen, wo sich das ablaufende Wasser sammelt und abtropft, mit Vorteil verwendbar.

Für die Halterung der Teströhrchen im umgekehrten Zustand mit der Öffnung nach unten können die verschiedensten Haltevorrichtungen verwendet werden, die die Teströhrchen sicher und beschädigungsfrei während des Waschvorgangs festhalten. Zu diesem Zwecke können Klemmen, z.B. Öffnungen mit über den Umfang angeordneten elastischen Klemmzungen, für die einzelnen Teströhrchen vorgesehen sein, es können aber beispielsweise auch mit rechenartigen Bügeln Reihen von Teströhrchen mit einem einzigen Befestigungsschritt in der Haltevorrichtung befestigt werden.

Eine schonende und einfache Befestigung der Teströhrchen in der Haltevorrichtung ergibt sich auch dann, wenn man die Teströhrchen in Öffnungen einsteckt, die mit Gummimanschetten versehen sind, die sich an die Teströhrchen anlegen und diese halten. Wenn derartige Gummimanschetten so ausgeführt werden, daß die Haltekraft pneumatisch verstärkt oder freigegeben wird, kann die Sicherheit auf einfache Weise erhöht werden. So können die Manschetten die Form von aufblasbaren ringförmigen Ballons aufweisen, die durch kurzes Beaufschlagen mit Druckluft gefüllt werden können und in diesem Zustand die Röhrchen auch bei der Verschwenkung mit der Öffnung nach unten sicher festhalten. Es ist anders herum auch möglich, die Röhrchen durch Anlegen von Unterdruck in der Haltevorrichtung festzusaugen, wobei ebenfalls geeignet gestaltete Dichtringe, vorzugsweise in Form von Gummimanschetten, zur Anwendung kommen können.

Nachfolgend wird die erfindungsgemäße Vorrichtung anhand eines Ausführungsbeispiels unter Bezugnahme auf Figuren noch näher erläutert.

Es zeigen:

Fig.1 eine Seitenansicht einer erfindungsgemäßen Vorrichtung mit einer Haltevorrichtung für fünfzig Teströhrchen sowie einer zugeordneten Sprühvorrichtung in teilweise geschnittener Darstellung;

Fig.2 eine Draufsicht auf die in Fig.1 gezeigte Sprühvorrichtung;

Fig.3 eine geschnittene Draufsicht auf den Boden der Sprühvorrichtung gemäß Fig.1 und Fig.2 mit dem System zur Zuführung des Spülwassers zu allen Sprühdüsen.

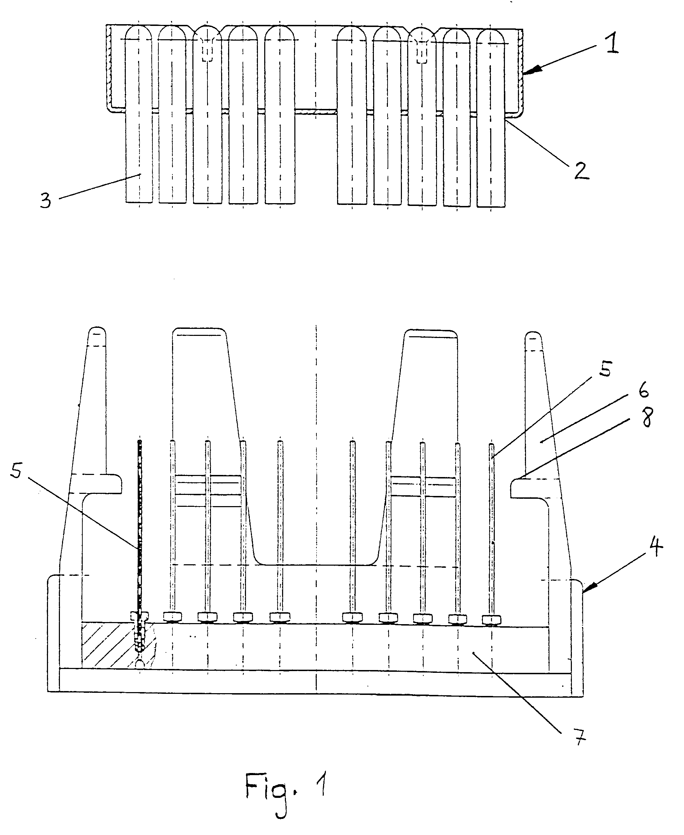

Nunmehr Bezug nehmend auf Fig.1 besteht eine erfindungsgemäße Vorrichtung gemäß einer einfachen und kostengünstig herzustellenden Ausführungsform aus einer Haltevorrichtung 1 und einer Sprühvorrichtung 4. Die Haltevorrichtung 1 weist die Form eines rechteckigen Kunststoffgestells in etwa der Form eines Kastens mit abgerundeten Kanten und nach oben weisender Grundfläche auf, in der als Halterungen 2 für Teströhrchen 3 fünfzig kreisförmige Öffnungen 2 ausgebildet sind, deren Durchmesser jeweils etwa dem Durchmesser der Teströhrchen 3 entspricht. Diese werden in den Öffnungen 2 festgeklemmt, wobei vorzugsweise um die Öffnungen 2 herum ein Kranz von elastischen Zungen ausgebildet ist, durch die die Teströhrchen 3 so festgeklemmt werden, daß die ganze Haltevorrichtung 1 mit den festgeklemmten Teströhrchen mit der Oberseite nach unten in die in Fig.1 dargestellte Stellung gedreht werden kann.

Die Sprühvorrichtung 4 weist einen mit einer Spülwasserzuführung versehenen Boden 7 auf, aus dem durch fünfzig Sprühdüsen 5 Spülwasser versprüht werden kann. Das "Spülwasser" kann in Einzelfällen einfaches Leitungswasser sein, ist jedoch vorzugsweise eine einem geeigneten Vorratsbehälter entnommene Waschlösung der eingangs genannten Art, wobei zur Gewährleistung eines konstanten Spülwasserdrucks zwischen dem Vorratsbehälter für die Waschlösung und der Sprühvorrichtung 4 eine geeignete Flüssigkeitspumpe vorgesehen ist. Der Boden 7 der Sprühvorrichtung 4 ist von einem erhabenen Auflagerand 6, vorzugsweise in Form vereinzelter Segmente, umgeben, der eine Führung und Halterung bildet, in die die Haltevorrichtung 1 mit nach unten weisender Oberseite paßgenau eingesetzt werden kann. Konsolen 8 legen die Höhe der Haltevorrichtung über dem Boden 7 der Sprühvorrichtung 4 fest.

Die Sprühdüsen 5 sind vorzugsweise so ausgebildet, daß sie in Verbindung mit dem Spülwasserzuleitungssystem in den Boden 7 eingeschraubt oder gesteckt werden können und ggf. auch auswechselbar sind. Die Sprühdüsen 5 sind vorzugsweise Röhrchen mit Sprühöffnungen an ihren oberen Enden und ragen bei aufgesetzter Haltevorrichtung 1 ins Innere der über ihnen angeordneten Teströhrchen.

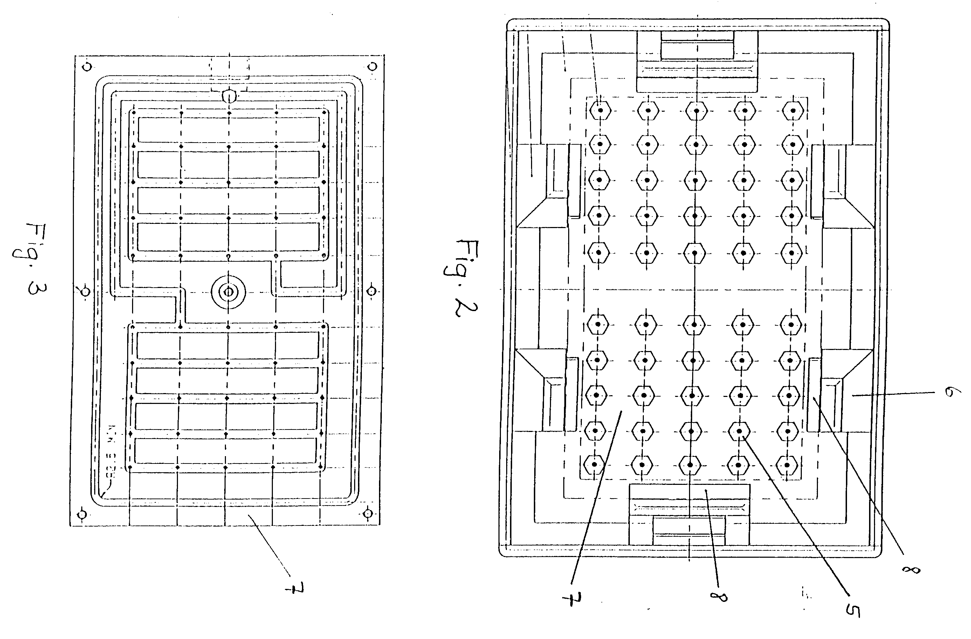

Um sicherzustellen, daß der Wasserdruck an allen fünfzig Sprühdüsen etwa gleich ist, wird ein mäanderartiges Leitungssystem (vgl. Fig.3) verwendet, das das Spülwasser von einem Wasserleitungsanschluß oder aus einer Zuleitung, durch die eine wäßrige Waschlösung mit einer Pumpe aus einem Vorratsbehälter für diese Waschlösung gefördert wird, gleichmäßig zu den einzelnen Düsen 5 der Sprühvorrichtung fördert.

Sowohl die Haltevorrichtung 1 als auch die Sprühvorrichtung 4 können aus Kunststoff hergestellt sein, z.B. aus einem klaren Polystyrol, und weisen auf diese Weise nur ein geringes Gewicht auf.

Bei der Benutzung werden die in der Haltevorrichtung 1 angeordneten Teströhrchen nach Abschluß der für die jeweilige Bestimmung erforderlichen Inkubation und nach dem Absaugen oder Abdekantieren der ursprünglichen flüssigen Phase mit ihren Öffnungen nach unten gedreht, und die Haltevorrichtung 4 wird in dieser Stellung auf die Konsolen 8 des Auflagerandes 6 aufgelegt bzw. in den Auflagerand 6 bis zum Anschlag an den Konsolen 8 eingesteckt.

In dieser Anordnung ragt je eine Sprühdüse 5 ins Innere eines jeden der Teströhrchen 3. Dann wird für die benötigte kurze Zeit das Spülwasser eingeschaltet, wodurch die Innenwände der Teströhrchen mit einem frei abfließenden Spülwassernebel gewaschen werden. Nach Beendigung des Waschschritts kann die Haltevorrichtung 1 wieder von der Sprühvorrichtung 4 gelöst werden, und die Teströhrchen können auf die durch den jeweiligen Test vorgegebene Weise vermessen werden.

Die nachfolgenden Vergleichsversuche belegen die Verbesserung der Testqualität durch das Waschen nach dem erfindungsgemäßen Verfahren.

**Vergleichsbeispiel 1**

In mit Klammern für die Fixierung der Teströhrchen versehenen Röhrchengestellen werden 50 Röhrchen unter Zuhilfenahme eines 5-fach Waschkammes, d.h. einer aus 5 nebeneinander angeordneten Sprühdüsen zusammengesetzten Sprühvorrichtung, die über die Gruppe der Röhrchen hinweggeführt wird und diese füllt, sowie einer Dispensette mit je 1 ml Berilux-Waschlösung gefüllt (im Handel mit dem Berilux TSH-ILMA der Firma Behring erhältliche Waschlösung). Anschließend wird die Waschlösung dekantiert. Diese Prozedur wird drei weitere Male wiederholt. Zum Schluß läßt man die Röhrchen 10 min auf Zellstoff abtropfen.

Die bei einer solchen Vorgehensweise erhältliche Spüleffektivität wird nach zwei weiter unten beschriebenen Verfahren ermittelt. Die Ergebnisse sind in der abschließenden Tabelle wiedergegeben.

**Vergleichsbeispiel 2**

In einem Gestell, wie es auch in Vergleichsbeispiel 1 verwendet wurde, werden Röhrchen mit je 2 ml TSH-Waschlösung (aus dem TSH-Kit Dynotest TSH der Firma Henning Berlin) gefüllt. Anschließend wird der Inhalt der Röhrchen mittels Wasserstrahlvakuum abgesaugt. Nach dreimaliger Wiederholung des Waschvorgangs läßt man auch diese Röhrchen 10 min auf Zellstoff abtropfen.

**Beispiel**

In einem Gestell, wie es in den Vergleichsbeispielen 1 und 2 verwendet wurde, werden die Teströhrchen angeordnet, wonach das Gestell um 180° nach unten gedreht und über einer Spülapparatur fixiert wird, die aus einem Spülkamm mit aufwärtsgerichteten Spüldüsen, die ins Innere der Teströhrchen hineinreichen, besteht. Zur Spülung wird insgesamt 5 s mit normalem Leitungswasser gespült (Durchfluß: 2 ml/s). Anschließend läßt man die Röhrchen auf Zellstoff abtropfen.

**Ermittlung der Spüleffektivität beim Spülen gemäß Vergleichsbeispiel 1, Vergleichsbeispiel 2 und dem Beispiel:**

**Meßreihe Nr. 1:** Als Maß für die Wirksamkeit der Spülung dient das Abspülen eines Farbstoffs.

In Röhrchen des Kits Dynotest-TSH der Firma Henning Berlin werden jeweils 1 ml einer Lösung pipettiert, die 2 % Lebensmittelfarbstoff Purpurrot Typ S3068, 1,5 % Rinderserumalbumin in 50 mM $NaPO_4$ enthielt und einen pH von 7,4 aufwies. Diese Röhrchen werden nach den Verfahren gemäß Vergleichsbeispiel 1, 2 sowie dem Beispiel gespült. Nach Abschluß der Spülung werden die Röhrchen mit je 1 ml Wasser befüllt, geschüttelt, und anschließend wird die UV-Absorption bei 506 nm gemessen (die gemessene UV-Absorption ist der Menge an in den Röhrchen zurückgebliebenem Farbstoff proportional).

Die Ergebnisse sind in der Tabelle wiedergegeben.

**Meßreihe Nr. 2:** Auswaschung eines lumineszenzmarkierten monoklonalen Antikörpers: Dynotest TSH-Röhrchen, wie sie auch in Meßreihe Nr. 1 verwendet wurden, wurden mit je 300 $\mu$l eines mit einem Akridiniumester markierten monoklonalen Antikörpers befüllt (Totalaktivität/Röhrchen: 15 000 000 Signale, in 5 % Rinderserumalbumin, 50 mM $NaPO_4$, pH 7,4).

Nach einer 20-minütigen Inkubationszeit werden die Röhrchen den Waschprozeduren gemäß Vergleichsbeispiel 1 und 2 sowie dem Beispiel unterworfen. Anschließend wird die verbleibende Lumineszenz in einem Berthold-Autoclinilumaten vermessen.Die Intensität der gemessenen Lumineszenz (Signale) ist der Menge der beim Waschen nicht entfernten lumineszenzmarkierten monoklonalen Antikörper proportional.

Die Ergebnisse sind in der nachfolgenden Tabelle zusammengefaßt:

Tabelle

| Waschverfahren | Meßreihe Nr. 1 (Farbstoffmethode) | |
| --- | --- | --- |
| | Mittelwert der bei 506 nm gemessenen UV-Absorption | Standard-abweichung |
| Vergleichsbeispiel 1 | 0,085 | 0,226 (n=100) |
| Vergleichsbeispiel 2 | 0,364 | 0,490 (n=100) |
| Beispiel | 0,002 | 0,002 (n=100) |

Tabelle

| Waschverfahren | Meßreihe Nr. 2 (Lumineszenzmethode) | |
| --- | --- | --- |
| | Lumineszenz-mittelwert (Signale) | Standard-abweichung |
| Vergleichsbeispiel 1 | 11572 | 3309 (n=100) |
| Vergleichsbeispiel 2 | 20213 | 3228 (n=100) |
| Beispiel | 4066 | 439 (n=100) |

Die Ergebnisse der obigen Tabelle zeigen, daß bei dem Waschen gemäß Beispiel, d.h. beim Spülen der Röhrchen mit nach unten weisenden Öffnungen, das Waschresultat wesentlich besser ist als bei den Vergleichsbeispielen. Die Verbesserung des Waschresultats ist nicht nur anhand der geringeren, im Röhrchen zurückgebliebenen Substanzmengen zu erkennen, sondern vor allem auch an den niedrigeren Variationskoeffizienten, die den Grad der Reproduzierbarkeit widerspiegeln.

Berücksichtigt man, daß der Zeitbedarf bei den Vergleichsbeispielen 1 und 2 jeweils etwa 30 s beträgt, während der Zeitbedarf gemäß Beispiel nur 5 s beträgt, ist die Überlegenheit des erfindungsgemäßen Waschverfahrens klar zu erkennen.

**Patentansprüche**

1. Verfahren zum Waschen von Trägern mit immobilisierten Reaktionsprodukten oder Reaktionspartnern, die bei immundiagnostischen Bestimmungsverfahren gebildet oder verwendet werden, insbesondere zur Durchführung der Waschschritte bei immundiagnostischen Verfahren zur Konzentrationsbestimmung von Biomolekülen wie Antikörpern, Antigenen und Haptenen in flüssigen Medien unter Verwendung von mit immobilisierten spezifischen Bindungspartnern für diese Biomoleküle oder mit immobilisierten Formen dieser Biomoleküle beschichteten festen Trägern, auf denen immobilisierte Immunkomplexe gebildet werden, oder bei der Herstellung derartiger Träger unter Beschichtung aktivierter unbeschichteter Träger mit derartigen spezifischen Bindungspartnern bzw. Formen der zu bestimmenden Biomoleküle,

   **dadurch gekennzeichnet, daß**

   man die Träger in dem oder den Waschschritt(en) so mit der Waschflüssigkeit behandelt, daß die Waschflüssigkeit während des Waschens frei von den Trägern mit den immobilisierten Immunkomplexen oder den immobilisierten spezifischen Bindungspartnern bzw. immobilisierten Formen der zu bestimmenden Biomoleküle abfließen kann.

2. Verfahren nach Anspruch 1, dadurch gekennzeichnet, daß man die Träger in Form von Teströhrchen oder Mikrotiterplatten mit ihren Öffnungen nach unten anordnet und die beschichteten Flächen mit einem Flüssigkeitsstrahl oder -nebel absprüht.

3. Verfahren nach Anspruch 2, dadurch gekennzeichnet, daß man eine Vielzahl von Teströhrchen oder Mikrotiterplatten gleichzeitig wäscht, wobei jedem Teströhrchen oder jeder Öffnung der Mikrotiterplatte eine eigene Sprühvorrichtung zugeordnet ist.

4. Verfahren nach Anspruch 2, dadurch gekennzeichnet, daß man eine Vielzahl von Teströhrchen oder Mikrotiterplatten bei einem einzigen Waschvorgang dadurch wäscht, daß man eine bewegliche Sprühvorrichtung verwendet, die die einzelnen Teströhrchen oder Öffnungen der Mikrotiterplatten nacheinander wäscht.

5. Verfahren nach einem der Ansprüche 1 bis 4, dadurch gekennzeichnet, daß man die von den Trägern ablaufende Waschflüssigkeit so ableitet bzw. sammelt, daß die aus einem Teströhrchen bzw. einer Öffnung einer Mikrotiterplatte ablaufende Waschflüssigkeit nicht mit den beschichteten Wänden eines anderen Teströhrchens bzw. einer anderen Öffnung einer Mikrotiterplatte in Berührung kommt.

6. Verfahren nach Anspruch 5, dadurch gekennzeichnet, daß man eine Sprühvorrichtung verwendet, bei der die Sprühdüse mit einer Leitung zum Sammeln des ablaufenden Waschwassers kombiniert ist.

7. Verfahren nach Anspruch 6, dadurch gekennzeichnet, daß man eine Sprühvorrichtung verwendet, bei der die Sprühdüse konzentrisch in der Leitung zum Sammeln des ablaufenden Waschwassers angeordnet ist und daß die Öffnung dieser Leitung am Rand des Teströhrchens bzw. der Öffnung der Mikrotiterplatte in Anlage gebracht werden kann.

8. Vorrichtung zur Durchführung des Verfahrens nach einem der Ansprüche 1 bis 7, gekennzeichnet durch eine Haltevorrichtung (1) mit Halterungen (2) für wenigstens ein Teströhrchen (3) bzw. eine Mikrotiterplatte, die mit dem mindestens einen Teströhrchen (3) bzw. der mindestens einen Mikrotiterplatte in einer Stellung mit nach oben weisenden Öffnungen und in einer Stellung mit nach unten weisenden Öffnungen anordenbar ist und in der die Teströhrchen (3) bzw. Mikrotiterplatten gegen ein Herausrutschen aus der Halterung (3) gesichert sind, sowie eine Sprühvorrichtung (4) mit mindestens einer Sprühdüse (5) zur Abgabe eines Flüssigkeitsstrahls oder -nebels in die mit den Öffnungen nach unten angeordneten Teströhrchen (3) bzw. Mikrotiterplatten.

9. Vorrichtung nach Anspruch 8, dadurch gekennzeichnet, daß die Haltevorrichtung (1) ein Kunststoffgestell mit Öffnungen (2) für eine Vielzahl von Teströhrchen (3) ist, die von um die Öffnungen (2) angeordneten elastischen Klemmzungen gehalten werden, und daß die Sprühvorrichtung (4) mit einem Auflagerand (6) für das Kunststoffgestell versehen ist, in den das Kunststoffgestell mit nach unten weisenden Öffnungen der Teströhrchen (3) so einsetzbar ist, daß der (die) von der (den) Sprühdüse(n) (5) abgegebene(n) Flüssigkeitsstrahl(en) in die Öffnungen der Teströhrchen (3) abgegeben wird (werden).

10. Vorrichtung nach Anspruch 8, dadurch gekennzeichnet, daß die Haltevorrichtung, gegebenenfalls zusammen mit der mindestens einen Sprühdüse, um eine waagerechte Drehachse oder in einer Führung um 180° verschwenkbar ist.

11. Vorrichtung nach Anspruch 8, 9 oder 10, dadurch gekennzeichnet, daß sie eine solche Anzahl von Sprühdüsen (5) aufweist, die der Anzahl der Halterungen für die Teströhrchen (3) bzw. der Anzahl der Öffnungen in den Mikrotiterplatten entspricht.

12. Vorrichtung nach Anspruch 8, 9 oder 10, dadurch gekennzeichnet, daß sie eine bewegliche Sprühvorrichtung aufweist, die schrittweise zu den Positionen der Halterungen für die Teströhrchen bzw. den Öffnungen der Mikrotiterplatten bewegbar ist.

13. Vorrichtung nach Anspruch 12, dadurch gekennzeichnet, daß die Bewegung der Sprühvorrichtung durch ein Programm steuerbar ist.

14. Vorrichtung nach einem der Ansprüche 8 bis 13, dadurch gekennzeichnet, daß sie eine Einrichtung zum Sammeln der ablaufenden Waschflüssigkeit aufweist.

15. Vorrichtung nach Anspruch 14, dadurch gekennzeichnet, daß die Einrichtung zum Sammeln der ablaufenden Waschflüssigkeit diese Waschflüssigkeit für jedes Teströhrchen bzw. jede Öffnung der Mikrotiterplatte getrennt sammelt, bevor die Waschflüssigkeit anschließend gegebenenfalls vereinigt und abgeführt wird.

16. Vorrichtung nach einem der Ansprüche 8 bis 15, dadurch gekennzeichnet, daß jede der mindestens einen Sprühdüsen mit einer Ableitung für die ablaufende Waschflüssigkeit so kombiniert ist, daß die gesamte aus einer Sprühvorrichtung abgegebene Waschflüssigkeit in dieser Ableitung gesammelt wird.

17. Vorrichtung nach Anspruch 16, dadurch gekennzeichnet, daß die Sprühvorrichtung mindestens eine Sprühdüse aufweist, die konzentrisch in einer rohrförmigen Ableitung für die Waschflüssigkeit angeordnet ist, wobei der Rand der Öffnung der Ableitung am Rand der Öffnung des Teströhrchens bzw. einer Öffnung der Mikrotiterplatte zur Anlage gebracht werden kann.

18. Vorrichtung nach Anspruch 17, dadurch gekennzeichnet, daß die Sprühdüse aus der Ableitung herausragt.

19. Vorrichtung nach einem der Ansprüche 8 bis 18, dadurch gekennzeichnet, daß die Haltevorrichtung als Halterungen Klemmvorrichtungen zur individuellen oder gruppenweisen Befestigung der Teströhrchen bzw. Mikrotiterplatten aufweist.

20. Vorrichtung nach Anspruch 19, dadurch gekennzeichnet, daß die Haltevorrichtung als Halterungen für die Teströhrchen Öffnungen mit an den Teströhrchen anliegenden elastischen Manschetten aufweist.

21. Vorrichtung nach Anspruch 20, dadurch gekennzeichnet, daß die Manschetten Gummimanschetten sind, die pneumatisch an den Teströhrchen zur Anlage gebracht werden können.

22. Vorrichtung nach Anspruch 21, dadurch gekennzeichnet, daß die Gummimanschetten aufblasbar sind.

23. Vorrichtung nach Anspruch 21, dadurch gekennzeichnet, daß die Gummimanschetten so ausgebildet sind, daß sie sich unter dem Einfluß eines in der Haltevorrichtung erzeugten Unterdrucks fest an die Oberflächen der Teströhrchen anlegen.

Fig. 1

Fig. 2

Fig. 3